# EUROPEAN PATENT APPLICATION

(11) **EP 4 574 833 A1**
(43) Date of publication of application: **25.06.2025**
(21) Application number: 24221845.1
(22) Date of filing: 19.12.2024
(51) Int. Cl.: C07H 1/02, C07H 21/00, C07H 21/02

(54) **SYNTHESIZING A NUCLEIC ACID OR NUCLEIC ACID ANALOGUE**

(30) Priority: 22.12.2023 EP 23220036
(71) Applicant: Imec VZW, 3001 Leuven (BE)
(72) Inventor: HOELZ, Kathrin, 3001 Leuven (BE)
(74) Representative: Winger

(57) **Abstract**

In a first aspect, the present invention relates to a method for synthesizing a nucleic acid or nucleic acid analogue, comprising: a) providing a root fragment of the nucleic acid or nucleic acid analogue, b) providing a further fragment of the nucleic acid or nucleic acid analogue, c) phosphitylating either the root fragment or the further fragment, and d) binding the further fragment to the root fragment by a phosphoramidite coupling; wherein the root fragment and the further fragment each have a length of at least 5 monomers, preferably at least 10 monomers, more preferably at least 20, yet more preferably at least 30, still yet more preferably at least 50.

## Description

### Technical field of the invention

The present invention relates to synthesizing a nucleic acid or nucleic acid analogue, and in particular wherein the synthesis involves phosphoramidite coupling.

### Background of the invention

Phosphoramidite chemistry is widely used to chemically synthesize (oligo)nucleic acids such as DNA, RNA, other nucleic acids and their analogues. In this workflow, a new building block is added to the surface-tethered growing strand in a monomer addition cycle which exists of four essential steps: coupling, capping, oxidation and detritylation.

In the coupling step a covalent bond is formed between the incoming phosphoramidite and the immobilized nucleic acid strand. Since the stepwise coupling efficiency of a monomer addition is below 100%, a certain percentage of strands remains unreacted. To prevent those unreacted strands from growing further and resulting in incorrect sequences, they are capped. This allows the separation of full-length products from truncated fragments.

However, due to the finite and compounding stepwise coupling yield, the length of the oligonucleotides that can be achieved using conventional phosphoramidite chemistry is limited, with a length of roughly up to 100-mers for DNA and 60-mers for RNA. With highly optimized protocols, oligonucleotide lengths of up to 200-mers for DNA and up to 120-mers for RNA can sometimes be reached but even these remain fairly short. Nonetheless, the area of applications for various oligonucleotides is growing rapidly; especially since the development of mRNA-based vaccines, which entail a high demand for RNA oligonucleotides of significantly longer length. The current workflow for producing oligonucleotides of such lengths is very labour-intensive and can easily take up several weeks.

US2021047361A1 sought to overcome these challenges and disclosed a method in which- rather than capping unreacted strands and prevent them from growing further-all the strands bear a 5'-OH-thermally cleavable protecting group which can be selectively removed by heating selected sites of the substrate. Upon selective thermal deprotection, a nucleoside 3'-phosphoramidite or a di- or tri-nucleotide 3'-phosphoramidite-again bearing a 5'-OH protecting group-is then coupled onto the deprotected 5'-OH groups. However, this approach has its own inherent limitations and shortcomings.

There is thus still a need in the art for approaches to nucleic acid synthesis which address at least some of the issues outlined above.

### Summary of the invention

It is an object of the present invention to provide a good method for synthesizing a nucleic acid (analogue); preferably suited for synthesizing a long nucleic acid, such as at least 1 00-mers or 1000-mers. It is a further object of the present invention to provide a good device associated therewith. This objective is accomplished by methods and devices according to the present invention.

Rather than growing a nucleic acid (analogue) strand sequentially through the cyclic addition of monomers-or even di- or trimers-using phosphoramidite chemistry, it was surprisingly realized within the present invention that the above objective-and in particular alleviating the issue of the limited length that can practically be achieved due to the finite and compounding stepwise coupling yield-can be met by providing (e.g. synthesizing) nucleic acid fragments of a substantial length for example and then binding them together using phosphoramidite coupling. Indeed, assuming in a first approximation a constant stepwise coupling yield y, the sequential addition of monomers to a length of n has a total yield of *γ*^{*n*-1}. For a length n of 100 and a stepwise coupling yield y of 99%, this results in a total yield of only 37%; for a yield y of 98% only 14%; etc. Conversely, the parallel synthesis-using the same sequential addition of monomers-of two strands of length n followed by binding both strands together has a total yield of *γ*^{*n*-1} × *δ* , wherein *δ* is the yield of combining both strands (or more generally, for assembling m strands of length n-and assuming in first approximation also a constant combination yield *δ*-the total yield is *γ*^{*n*-1} × *δ*^{*m*-1}). Combining two strands of length 50 (to yield the same total length 100 as above), and even assuming a substantially lower yield *δ* of only 80%, for a yield y of 99% this results in a total yield of 49%; for a yield y of 98% still 30%; etc.

From the above, it will be clear that the optimal length for the nucleic acid (analogue) fragments before coupling them together generally depends on several factors, including the yield of synthesizing the individual fragments and the yield of combining them. Notwithstanding, typically a worthwhile improvement (compared to sequentially synthesizing the entire nucleic acid (analogue)) can already be achieved for fragments with a length of at least 5 monomers; with the gain increasing for longer fragments. While it is still typically beneficial over sequentially synthesizing the entire nucleic acid (analogue), for increasingly long fragments not only the yield of synthesizing them but also the yield of combining them may drop off significantly. However, this can in turn be alleviated by applying the approach of the present invention multiple times (optionally in (semi-)parallel manner), e.g. in a sequential or hierarchical manner. For example, four fragments A, B, C and D can be provided and combined either as: A+B→A-B, C+D→C-D, A-B+C-D→A-B-C-D (i.e. hierarchically); or as: A+B→A-B, A-B+C→ A-B-C, A-B-C+D→A-B-C-D (i.e. sequenctially).

Beyond the increase in practically achievable length (through increasing the total yield), this approach moreover allows for the different fragments to be synthesized in parallel (e.g. on different substrates; or on different areas of the same substrate), thereby also substantially decreasing the total time needed to synthesize the whole nucleic acid (analogue).

It is an advantage of embodiments of the present invention that the total yield of synthesizing the nucleic acid (analogue) can be increased. It is a further advantage of embodiments of the present invention that the length of the nucleic acid (analogue) which can be practically achieved can be increased.

It is an advantage of embodiments of the present invention that the root fragment and the further fragment can be provided (e.g. synthesized) in parallel. It is a further advantage of embodiments of the present invention that the total time needed to synthesize the nucleic acid (analogue) can be decreased.

It is an advantage of embodiments of the present invention that the approach can be applied multiple times, allowing for even longer nucleic acid fragments to be synthesized with maintaining relatively high yields.

It is an advantage of embodiments of the present invention that the method may include inherent purification, thereby obviating a dedicated purification step.

It is an advantage of embodiments of the present invention that the method can be adapted to accommodate different synthesis directions for the fragments.

It is an advantage of embodiments of the present invention that inadvertent reactions between fragments can be reduced or prevented.

It is an advantage of embodiments of the present invention that the nucleic acid (analogue) can be detached and collected for use in various applications.

It is an advantage of embodiments of the present invention that it can be performed in a relatively straightforward and economical fashion.

In a first aspect, the present invention relates to a method for synthesizing a nucleic acid or nucleic acid analogue, comprising: a) providing a root fragment of the nucleic acid or nucleic acid analogue, b) providing a further fragment of the nucleic acid or nucleic acid analogue, c) phosphitylating either the root fragment or the further fragment, and d) binding the further fragment to the root fragment by a phosphoramidite coupling; wherein the root fragment and the further fragment each have a length of at least 5 monomers, preferably at least 10 monomers, more preferably at least 20, yet more preferably at least 30, still yet more preferably at least 50.

In a second aspect, the present invention relates to a device for synthesizing a nucleic acid or nucleic acid analogue by the method according to any embodiment of the first aspect, comprising: i) a component configured for receiving the root fragment and the further fragment of the nucleic acid or nucleic acid analogue, ii) a component configured for phosphitylating either the root fragment or the further fragment, and iii) a component configured for binding the further fragment to the root fragment by a phosphoramidite coupling.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantially new and novel improvements, including departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

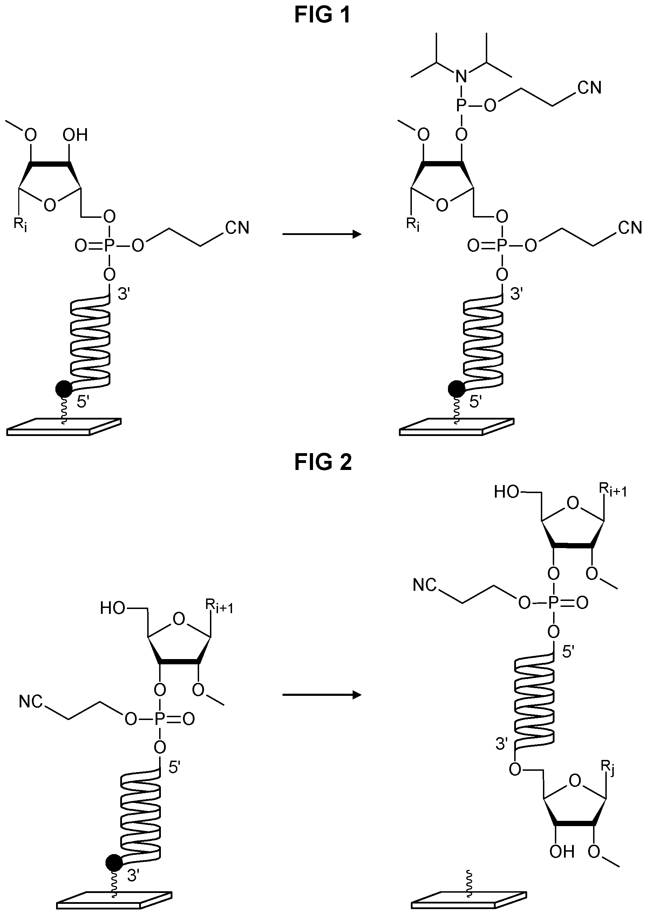

FIG 1 to FIG 9 schematically depict different reaction steps in various illustrative embodiments in accordance with the present invention.

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable with their antonyms under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. The term "comprising" therefore covers the situation where only the stated features are present and the situation where these features and one or more other features are present. Thus, the scope of the expression "a device comprising means A and B" should not be interpreted as being limited to devices consisting only of components A and B. It means that with respect to the present invention, the only relevant components of the device are A and B.

Similarly, it is to be noticed that the term "coupled", also used in the claims, should not be interpreted as being restricted to direct connections only. The terms "coupled" and "connected", along with their derivatives, may be used. It should be understood that these terms are not intended as synonyms for each other. Thus, the scope of the expression "a device A coupled to a device B" should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practised without these specific details. In other instances, well-known methods, structures and techniques have not been shown in detail in order not to obscure an understanding of this description.

The following terms are provided solely to aid in the understanding of the invention.

As used herein, and unless otherwise specified, `nucleic acid (analogue)' refers to a nucleic acid or nucleic acid analogue. As known in the art, a `nucleic acid analogue' is a structural analogue to (naturally occurring) nucleic acids, such as DNA (deoxyribonucleic acid) or RNA (ribonucleic acid). Where nucleic acids are chains of nucleotides, which are composed of three parts: a phosphate backbone, pentose sugar (deoxyribose or ribose) and nucleobase (adenine, cytosine, guanine, thymine or uracil); a nucleic acid analogue is a chain of nucleotide analogues, which may have any of those parts altered. Particular examples include analogues having a different sugar-also referred to as 'xeno nucleic acids' (XNA)-, and/or having modified or artificial nucleobases, and/or having modified backbones.

As used herein, and unless otherwise specified, when an end of a fragment is said to be rendered 'less reactive' to phosphoramidite coupling, it is meant that the end is less reactive to phosphoramidite coupling than if said end would bear an -OH group. Similarly, when the end of a fragment is said to be rendered 'unreactive' to phosphoramidite coupling, it is meant that the reaction speed of phosphoramidite coupling is at least 10 times lower than if said end would bear an -OH group; preferably at least 20 times, more preferably at least 100 times, yet more preferably at least 1000 times, still yet more preferably at least 10000 times, most preferably at least 100000 times.

In a first aspect, the present invention relates to a method for synthesizing a nucleic acid or nucleic acid analogue, comprising: a) providing a root fragment of the nucleic acid or nucleic acid analogue, b) providing a further fragment of the nucleic acid or nucleic acid analogue, c) phosphitylating either the root fragment or the further fragment, and d) binding the further fragment to the root fragment by a phosphoramidite coupling; wherein the root fragment and the further fragment each have a length of at least 5 monomers, preferably at least 10 monomers, more preferably at least 20, yet more preferably at least 30, still yet more preferably at least 50.

In embodiments, a ratio of the length of the further fragment to the length of the root fragment (length further fragment: length root fragment) may be from 0.1 to 10, preferably from 0.2 to 5, more preferably from 0.5 to 2, yet more preferably from 0.8 to 1.2, still yet more preferably from 0.9 to 1.1, most preferably from 0.95 to 1.05. The advantageous effect of the present invention on the efficiency (e.g. the total yield and/or speed) of the synthesis may typically be most pronounced if the root fragment and further fragment have a length which is at least in the same order of magnitude, and more preferably substantially similar (e.g. having a ratio from 0.8 to 1.2). Notwithstanding, depending on the embodiment, the further fragment(s) may be more easily purified (e.g. to remove erroneous fragments) than the root fragment(s). Accordingly, in such embodiments it may nonetheless prove beneficial to start from a comparatively shorter (thereby having a lower error-rate) root fragment. For example, the ratio of the length of the further fragment to the length of the root fragment may be from 1.05 to 10, preferably from 1.1 to 5, more preferably from 1.2 to 2.

In embodiments, the root fragment and the further fragment may each comprise at least two different monomers (e.g. two monomers having different nucleobases), preferably at least three different monomers, more preferably at least four different monomers.

In general, the root fragment and further fragment may be provided in step a and step b in any suitable way; for example, the root fragment and/or further fragment may be synthesized, separated from a mixture (e.g. from a biological sample), etc. Notwithstanding, step a may often comprise synthesizing the root fragment. Similarly, step b may often comprise synthesizing the further fragment. Synthesizing the root fragment and the further fragment may likewise be performed in any suitable way, such as by any suitable chemical, biochemical and/or biological process. Notwithstanding, the root fragment and/or the further fragment-preferably both-may in preferred embodiments be synthesized using phosphoramidite synthesis. In embodiments, the root fragment and/or the further fragment-preferably both-may be synthesized using a method comprising capping an erroneous fragment to prevent further chain elongation. Phosphoramidite synthesis is advantageously a well-established process for synthesizing new (i.e. not limited to copying existing strands) nucleic acid (analogue)s; moreover, it relatively straightforwardly allows to cap erroneous (e.g. unreacted) fragments to prevent further chain elongation. In embodiments wherein the root fragment and/or further fragment to be attached to a substrate via a linker (cf. infra), the substrate may be pre-functionalized with the linker (i.e. prior to attaching the fragment and/or starting the fragment synthesis), or the linker may be attached to substrate together with the fragment or as part of the fragment synthesis.

In embodiments, the root fragment and the further fragment may be synthesized concurrently (i.e. simultaneously, in parallel). In embodiments, the root fragment and/or the further fragment may be synthesized on a substrate. In preferred embodiments, the root fragment and the further fragment may be synthesized on a different substrate (or on separate areas of the same substrate). Alternatively, in embodiments wherein the further fragment can be detached from the substrate selectively with respect to the root fragment, the root fragment and the further fragment may be synthesized on the same substrate (e.g. in the same area). Detaching the further fragment from the substrate selectively with respect to the root fragment may be preformed in a way that is controlled by light, temperature or (electro)chemistry; for example using a reversible bond or a cleavable linker (cf. infra). Selectively releasing the further fragment may be performed on a fragment-by-fragment (i.e. individual control of each fragment release) or collective-by-collective (e.g. having different areas with a plurality of fragments in each and releasing all of the fragments in an area simultaneously).

Step c is typically performed to obtain a phosphoramidite. In embodiments, in step c the root fragment or further fragment may be phosphitylated while attached to a substrate. In embodiments, step c may be preformed in a same location (e.g. a same reaction chamber) as step d (i.e. *in situ*). For example, the root fragment may be phosphitylated while attached to the assembly substrate (cf. infra).

Compared to doing so in solution, phosphitylating the root fragment or further fragment while attached to a substrate advantageously contributes to preventing any inadvertent reaction between an already phosphitylated fragment and yet to be phosphitylated fragment. In embodiments, the root fragment or further fragment may be phosphitylated using a phosphitylating agent, such as chloro N,N,N',N'-tetraisopropyl phosphorodiamidite. In embodiments, the root fragment or further fragment may be phosphitylated using the phosphitylating agent in presence of a base and an activator (e.g. a triazole activator).

Typically, in step d the further fragment may be dispersed (e.g. dissolved or suspended) in a reaction medium (e.g. a solvent or buffer). This advantageously allows to easily contact and react the further fragment with the root fragment. In preferred embodiments, in step d the root fragment may be attached to a substrate (e.g. an assembly substrate). This advantageously allows to immobilize the root fragment-and thus also the nucleic acid (analogue) resulting from step d-and simultaneously protect the end of the root fragment which is not intended to couple with the further fragment (cf. infra). In other embodiments, in step d the root fragment and the further fragment may both be dispersed in the reaction medium.

The root fragment and the further fragment generally each have two ends, typically referred to as the 3' end (i.e. the 3' carbon of the pentose sugar at one extreme of the fragment) and the 5' end (i.e. the 5' carbon of the pentose sugar at the opposite extreme of the fragment). The 3' end is a secondary carbon while the 5' end is a primary carbon, as such the 5' end is typically more reactive due to steric hindrance and a lower stabilizing effect through the attached groups. Normally, the desired binding of the further fragment to the root fragment is moreover by coupling the 3' end of one fragment to the 5' end of the other fragment (i.e. not 3' to 3' or 5' to 5', as this would yield a nucleic acid (analogue) with a distorted directionality).

In a first type of embodiments therefore, the desired directionality of the binding of the root fragment and further fragment may be realized by using the inherent difference in reaction kinetics between the 3' end and the 5' end. More in particular, the root fragment may have its 5' end protected (i.e. rendered less reactive-preferably unreactive-to phosphoramidite coupling, e.g. through being attached through said end to the assembly substrate). Meanwhile the further fragment may have both ends reactive (i.e. not protected). Accordingly, with the 3' end of the root fragment phosphitylated, the more reactive 5' end of the further fragment will more readily bind through phosphoramidite coupling to the root fragment, thereby realizing the desired directionality. In this first type of embodiments, it may be less preferred to phosphitylate the further fragment-as opposed to the root fragment-, as-given that both ends of the further fragments are reactive-this could easily lead to inadvertent reactions between already phosphitylated fragments and yet to be phosphitylated fragments, thereby reducing the overall yield of the desired nucleic acid (analogue).

Alternatively, in a second type of embodiments, in step d the root fragment and the further fragment may each have an end (i.e. the 3' or 5' end) protected (i.e. rendered less reactive-preferably unreactive-to phosphoramidite coupling). For the root fragment, this may for example involve being attached through said end to the assembly substrate. For the further fragment-and as an alternative to the substrate for the root fragment-, this may involve the end being functionalized with a protective group. To that end, in some embodiments, the root fragment and/or the further fragment may-prior to step d (e.g. during their synthesis)-be at one end attached to the substrate by a linker, the linker being such that said fragment can be detached from the substrate while leaving a protective group on said end rendering the end less reactive-preferably unreactive-to phosphoramidite coupling. For example, the fragment may be-through the to-be-protected end-attached either to a linker which is in turn attached to the substrate by a reversible bond, or to a cleavable linker. The reversible bond may be for instance be a thermo-, pH-, redox- and/or photolabile bond; or, similarly, the cleavable linker may be a thermo-, pH-, redox- and/or photolabile linker. Accordingly, upon detaching or cleaving the linker, at least a portion of the linker remains attached to and protects the end of the fragment. Alternatively, the end may be protected by directly functionalizing said end with the protective group (e.g. while the fragment is attached to a substrate through the opposite end).

Regarding, the directionality of the fragments in the second type of embodiments, to realize a 3' to 5' (or 5' to 3') binding of the further fragment to the root fragment, the root fragment and further fragment should generally be protected at opposite ends. For example, where the root fragment is protected through its attachment to the assembly substrate: the root fragment may be attached to the assembly substrate through its 5' end and the further fragment may comprise a protective group at its 3' end, or the root fragment may be attached to the assembly substrate through its 3' end and the further fragment may comprise a protective group at its 5' end. Accordingly, the synthesis direction of the further fragment may also typically depend on the orientation of the root fragment and the way the protection of the further fragment is realized: if the protected end is the end through which the further fragment was attached to the synthesis substrate, the synthesis direction (e.g. 3' to 5') of the further fragment is typically opposite to the orientation (e.g. 5' to 3', the 5' end being attached to the assembly substrate) of the root fragment (and thus, if the root fragment is synthesized on the assembly substrate, the synthesis direction of the root fragment and further fragment may typically be opposite). More specifically, the further fragment may in such a case be synthesized on a synthesis substrate: in a 3' to 5' direction if the root fragment is attached to the assembly substrate through its 5' end, or in a 5' to 3' direction if the root fragment is attached to the assembly substrate through its 3' end. Conversely, if the protected end is the end away from the substrate, the synthesis direction of the further fragment is typically equal to the orientation of the root fragment.

In embodiments wherein the root fragment and/or the further fragment-preferably both-are synthesized using a method comprising capping an erroneous fragment to prevent further chain elongation (cf. supra), providing the root fragment and the further fragment with the end rendered less reactive-preferably unreactive-to phosphoramidite coupling may be performed such that erroneous fragments are less reactive-preferably unreactive-to phosphoramidite coupling at both ends. Since such capped and/or protected fragments are not readily available for binding with another fragment, an inherent purification is advantageously achieved without needing a dedicated purification step.

In embodiments wherein the root fragment is attached to the assembly substrate, the method may further comprise-after step d-: detaching the nucleic acid (analogue) from the assembly substrate. This can for instance be realized by the root fragment being attached i) directly to the assembly substrate or to a linker on the assembly substrate by a reversible bond, or ii) to a linker attached to the assembly substrate by a reversible bond or to a cleavable linker. As previously described, the reversible bond may be a thermo-, pH-, redox- and/or photolabile bond; or, similarly, the cleavable linker may be a thermo-, pH-, redox- and/or photolabile linker. Detaching the nucleic acid (analogue) advantageously allows to collect it and use elsewhere (in the same device or in another location) for its intended application.

In embodiments wherein the root fragment and/or further fragment is protected at one end by a protective group, the method may further comprise-after step d-: removing the protective group. Removing the protective group advantageously allows to recover the reactive end, which may be needed or useful for the synthesized nucleic acid (analogue)'s intended application; including using it as a new root fragment or further fragment (cf. infra). Note that where the protective group was provided as (a portion of) a linker remaining attached to the fragment after detaching or cleaving the linker, removing the protective group typically entails that the linker was detachable and/or cleavable at two locations, each with its own distinct trigger (e.g. one being thermolabile and the other photolabile, or both being photolabile but at different wavelengths).

In embodiments, after step d the root fragment extended with the further fragment may become a new root fragment or a new further fragment, and wherein step b to d may be repeated at least 1 time; preferably at least 2 times, more preferably at least 5 times, yet more preferably at least 10 times, most preferably at least 20 times.

In embodiments, any feature of any embodiment of the first aspect may independently be as correspondingly described for any embodiment of any of the other aspects.

In a second aspect, the present invention relates to a device for synthesizing a nucleic acid or nucleic acid analogue using (e.g. by) the method according to any embodiment of the first aspect, comprising: i) a component for (e.g. configured for) receiving the root fragment and the further fragment of the nucleic acid or nucleic acid analogue, ii) a component for (e.g. configured for) phosphitylating either the root fragment or the further fragment, and iii) a component for (e.g. configured for) binding the further fragment to the root fragment by a phosphoramidite coupling.

In embodiments, component i may be for synthesizing the root fragment, the further fragment or both. In embodiments, component i may be for synthesizing the root fragment, and the device may further comprise: iv) a component for synthesizing the further fragment.

In embodiments, the device may further comprise: iv) a component for detaching the synthesized nucleic acid (analogue).

In embodiments, one or more of the components may be a single component (i.e. they need not be distinct). For example, component i, ii, iii and-if present-iv may be a single component (e.g. a reaction chamber) in which the root fragment (or further fragment) is provided (e.g. synthesized), phosphitylated, bound to the further fragment (or root fragment) and optionally the resulting nucleic acid (analogue) is detached.

In embodiments, the component i for synthesizing the root fragment and/or the further fragment may comprise a plurality of synthesis sites (or areas; cf. supra) wherein each synthesis site is configured for synthesizing at least one root fragment or at least one further fragment. In embodiments, each synthesis site may be configured for synthesizing a plurality of root fragments or further fragments. Advantageously, the simultaneous synthesis of the same type of (e.g. sequentially identical) root fragments or further fragments can thereby be multiplexed.

In embodiments, the component i for synthesizing the root fragment and/or the further fragment may comprise a plurality of synthesis sites (or areas; cf. supra) wherein at least two different types of (e.g. being sequentially different) root fragments are synthesized on two different synthesis sites simultaneously, and/or at least two different types of further fragments are synthesized on two different synthesis sites simultaneously. Advantageously, the simultaneous synthesis of different types of (e.g. being sequentially different) root fragments and/or further fragments can be multiplexed. Each of the synthesis sites can be configured to be addressed individually by the device (e.g. being activated individually by light, temperature and/or (electro)chemistry).

In embodiment, at least one synthesis site may comprise an electrode configured to electrochemically synthesize the root fragment or further fragment. In embodiments, each synthesis site may comprise such an electrode.

In embodiments, any feature of any embodiment of the second aspect may independently be as correspondingly described for any embodiment of any of the other aspects.

The invention will now be described by a detailed description of several embodiments of the invention. It is clear that other embodiments of the invention can be configured according to the knowledge of the person skilled in the art without departing from the true technical teaching of the invention, the invention being limited only by the terms of the appended claims.

### Example 1

A first example of a nucleic acid (analogue) synthesis in accordance with the present invention is schematically depicted in FIG 1, FIG 2, FIG 3 and FIG 9.

This example starts with the provision of the root fragment (FIG 1, left side); here attached to an assembly substrate. This root fragment can for instance be obtained by attaching a starting fragment (e.g. a nucleoside or (oligo)nucleotide) to the assembly substrate and elongating it *in situ* by sequentially adding (e.g. using phosphoramidite coupling) nucleosides or (oligo)nucleotides. Alternatively or complementarily, the root fragment may be (at least partially) formed elsewhere and subsequently attached to the assembly substrate.

Next, the root fragment is phosphitylated while attached to the assembly substrate (FIG 1).

In parallel, a further fragment is provided (FIG 2). Similar to the root fragment, the further fragment can for instance be obtained by attaching a starting fragment (e.g. a nucleoside or (oligo)nucleotide) to a synthesis substrate and elongating it by sequentially adding (e.g. using phosphoramidite coupling) nucleosides or (oligo)nucleotides. Subsequently, the further fragment is detached from the synthesis substrate. This can for instance be achieved through the further fragment being attached directly to the synthesis substrate or to a linker on the synthesis substrate by a reversible bond, such as a thermo-, pH- and/or photolabile bond.

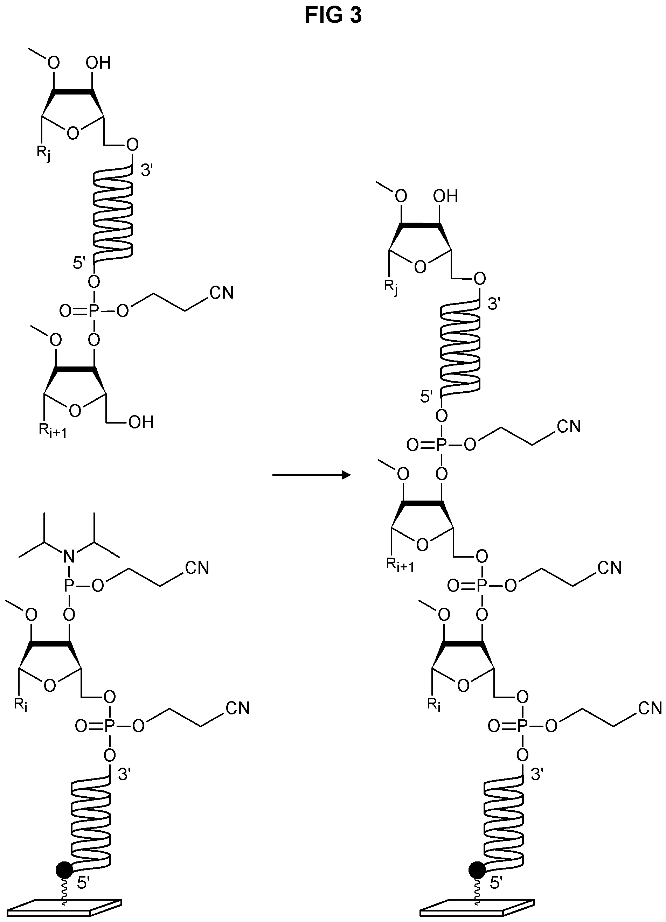

After being detached from the synthesis substrate, the further fragment is brought near the phosphitylated root fragment and allowed to react in a phosphoramidite coupling, thereby binding the further fragment to the root fragment (FIG 3).

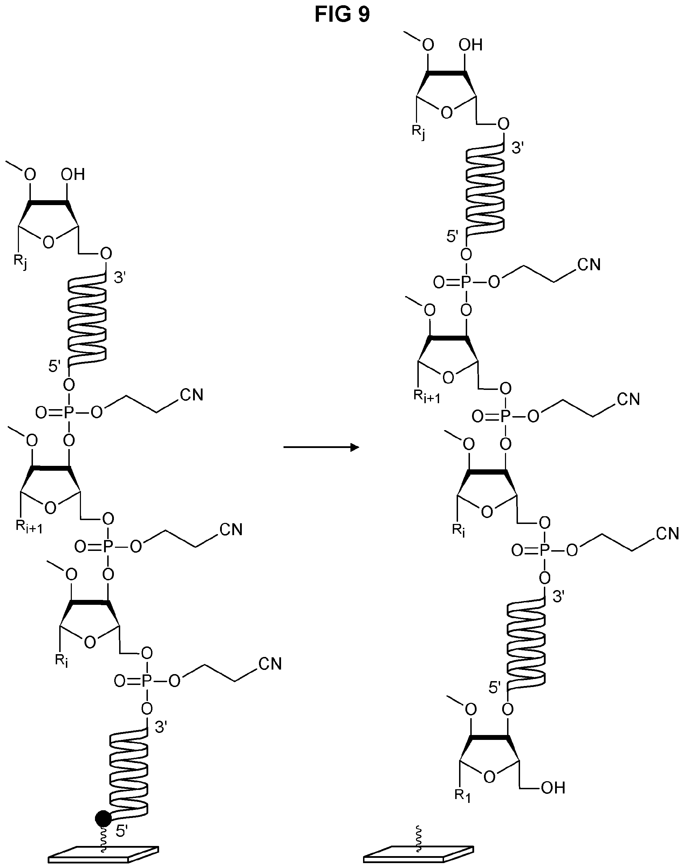

Optionally, also the attachment of the root fragment to the assembly substrate is reversible, so that the synthesized nucleic acid (analogue) can be detached from the assembly substrate (FIG 9). As desired-e.g. in view for the further use of the nucleic acid (analogue)-, this may involve the root fragment being attached i) directly to the assembly substrate or to a linker on the assembly substrate by a reversible bond, or ii) to a linker attached to the assembly substrate by a reversible bond or to a cleavable linker. Here, the reversible bond may again be a thermo-, pH-, redox- and/or photolabile bond; or, similarly, the cleavable linker may be a thermo-, pH-, redox- and/or photolabile linker.

After the above synthesis, the obtained nucleic acid (analogue) may be used in a variety of applications, including being used as a root fragment or further fragment in a new synthesis in accordance with the present invention.

Note that as depicted, the root fragment is attached to the assembly substrate via its 5' end; entailing that-if the root fragment is (at least partially) formed *in situ*-this involves a reverse (i.e. 5' to 3') synthesis. Although not strictly necessary, this is the preferred orientation for the root fragment in the present example, where both the 3' and 5' end of the further fragment are in principle free to react. Indeed, knowing that the 5' end of the further fragment is typically more reactive than its 3' end (cf. supra), this orientation sets up the root fragment to couple with the further fragment in the desired 3' (of the root fragment) to 5' (of the further fragment) coupling. If both ends are indeed 'free' (i.e. are reactive, e.g. in the form of bearing -OH group), the synthesis orientation of the further fragment can be selected as desired. Notwithstanding, if providing the further fragment comprises capping erroneous (e.g. unreacted) strands to prevent them from growing further-as is commonly done in phosphoramidite synthesis (cf. background)-it may be preferred to synthesize the further fragment in the forward (i.e. 3' to 5') direction. This will result in the erroneous strands being capped off at the more reactive 5' end, thereby deterring these erroneous strands from coupling with a root fragment more than if the less reactive 3' was capped off.

### Example 2

A second example of a nucleic acid or nucleic acid (analogue) synthesis in accordance with the present invention is schematically depicted in FIG 1, FIG 4, FIG 6, FIG 8 and FIG 9.

This example starts the same as Example 1, with the provision of the root fragment attached to the assembly substrate and subsequent phosphitylation of the root fragment (FIG 1).

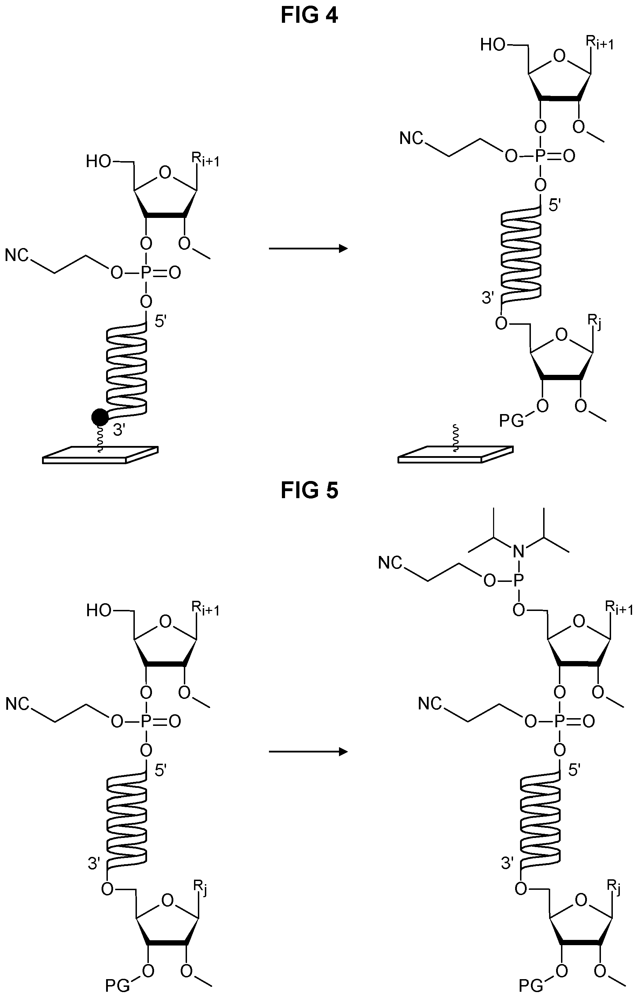

Also the provision of the further fragment is similar to that of Example 1, but with the difference that after detaching the further fragment from the synthesis substrate, one end (3' or 5') is protected by bearing a protective group (PG)-rendering said end less reactive (preferably unreactive) to phosphoramidite coupling (compared to the end bearing an -OH group)-while the other end remains unaltered (e.g. as a reactive end). As depicted in FIG 4, this can be realized by attaching the further fragment to the synthesis substrate in such a way that after detaching the further fragment the protective group is formed (or remains) at the end through which the further fragment was attached to the synthesis substrate. For instance, the further fragment may be attached to the synthesis substrate by a cleavable or detachable linker, such that at least a portion of the linker remains attached to the further fragment upon cleaving/detaching the linker. Alternatively, the protection at one end of the further fragment may also be provided simply by functionalizing said end with the protective group. Preferably, this can be done while the further fragment is still attached to the synthesis substrate (not depicted), thereby ensuring that only one end is protected (and that this protected end is the same for each further fragment). In this case, the protected end is then the end away from the substrate (i.e. opposite to what is depicted in FIG 4) and the other end is freed by detaching the further fragment as in Example 1.

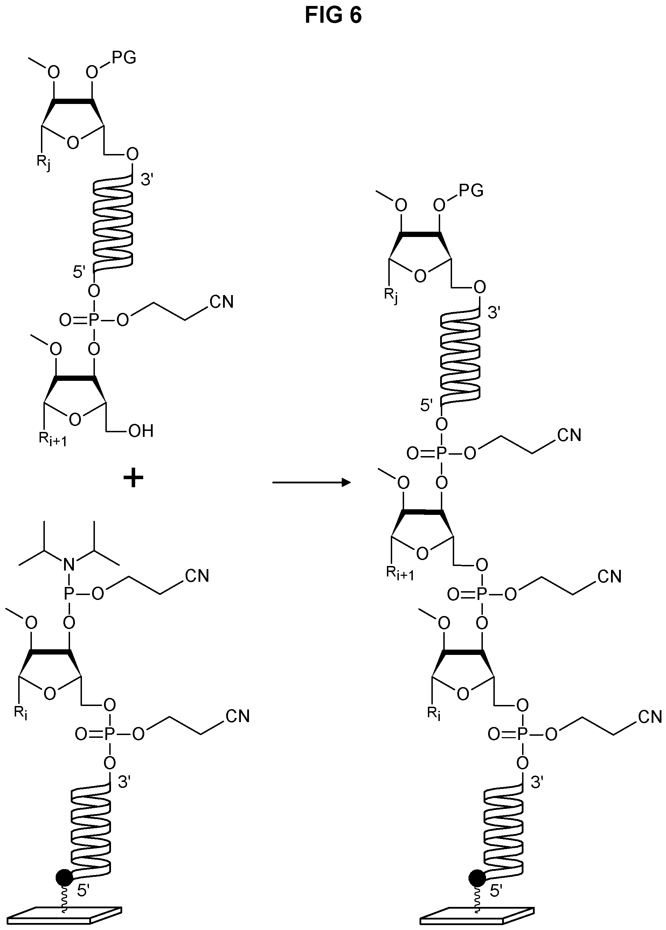

Next, the detached further fragment is again brought near the phosphitylated root fragment and allowed to react in a phosphoramidite coupling, thereby binding the further fragment to the root fragment (FIG 6).

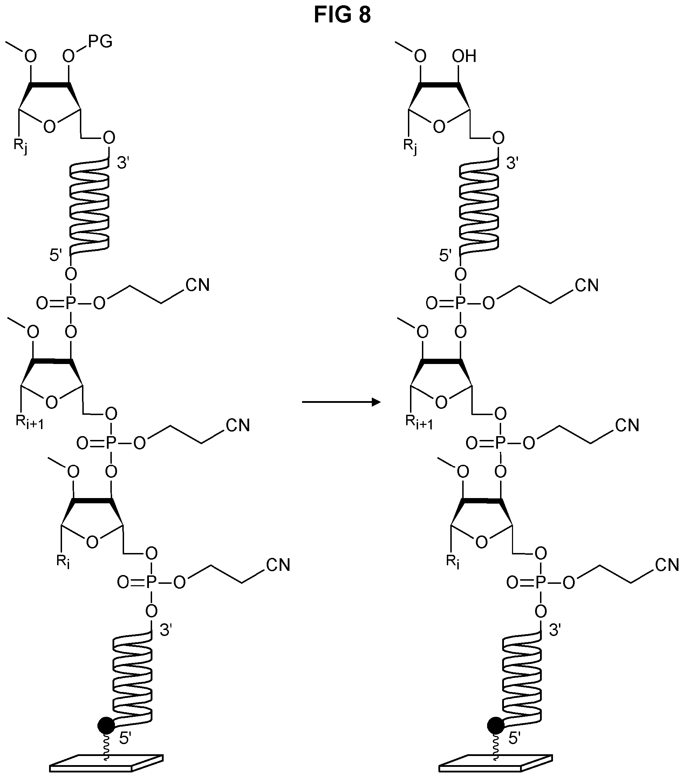

Preferably, the protection of one end of the further fragment is removable. FIG 8 for instance shows the protective group being removed-thereby recovering a reactive end-after coupling the further fragment to the root fragment, while the resulting nucleic acid (analogue) is still attached to the assembly substrate. Notwithstanding, it will be clear that the protection could also be removed after detaching the nucleic acid (analogue). Removing the protection is particularly useful when the protected end should be rendered reactive again; for instance, because it is to participate in a new synthesis in accordance with the present invention.

Finally, the attachment of the root fragment to the assembly substrate is optionally again reversible, so that the synthesized nucleic acid (analogue) can be detached from the assembly substrate (FIG 9).

Note that as depicted, the root fragment is here also attached to the assembly substrate via its 5' end. However, since-in contrast to Example 1-the coupling between the root fragment and the further fragment does here not rely on a difference in reaction kinetics between a free 3' and a free 5' end, the opposite orientation is in the present example equally possible. Depending on the orientation of the root fragment and the way the protection is realized (i.e. at the end through which the further fragment was attached to the synthesis substrate or at the end away from the substrate), the further fragment can be synthesized in the forward or reverse direction. Generally though, because the further fragment and root fragment are typically to be coupled in a 3' to 5' (or 5' to 3') fashion, if the protected end is the end through which the further fragment was attached to the synthesis substrate, the synthesis direction (e.g. 3' to 5') of the further fragment is typically opposite to the orientation (e.g. 5' to 3', the 5' end being attached to the assembly substrate) of the root fragment; and *vice versa:* if the protected end is the end away from the substrate, the synthesis direction of the further fragment is typically equal to the orientation of the root fragment. The above notwithstanding, if providing the further fragment comprises capping erroneous strands to prevent them from growing further, it may be preferred to protect the end through which the further fragment was attached to the synthesis substrate-and thus to synthesize the further fragment in the direction opposite of the orientation of the root fragment. This will result in the erroneous strands being capped off at the non-protected end, thereby rendering these erroneous strands unreactive at both ends and thus preventing them from coupling with a root fragment.

### Example 3

A third example of a nucleic acid or nucleic acid (analogue) synthesis in accordance with the present invention is schematically depicted in FIG 4, FIG 5, FIG 7, FIG 8 and FIG 9.

This example is similar to Example 2, but with the difference that not the root fragment but the further fragment is phosphitylated priorto coupling both. Accordingly, the root fragment is provided and left as is (FIG 1, left side), and the further fragment is provided and outfitted with a protective group at one end (e.g. FIG 4); both as previously described.

Next, the further fragment is phosphitylated. As depicted in FIG 5, this is performed after the further fragment was detached from the synthesis substrate; however, where the protected end is the end through which the further fragment was attached to the synthesis substrate, the phosphitylation may already be performed while the further fragment is still attached to the synthesis substrate. The latter may even be preferred in that case, as phosphitylating the further fragments in solution-depending on the speed of completion-risks that a yet to be phosphitylated further fragment would inadvertently couple (in a 3' to 3' or 5' to 5' fashion) with an already phosphitylated further fragment; which risk can be (at least partially) mitigated by performing the phosphitylation while the further fragments are still attached to the substrate.

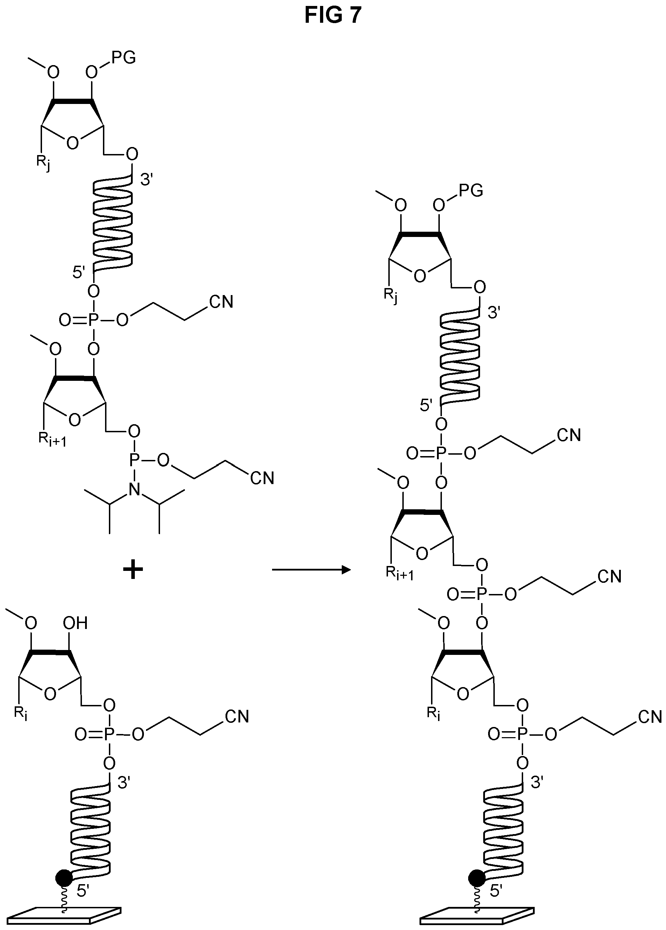

Subsequently, the phosphitylated further fragment is brought near the root fragment and allowed to react in a phosphoramidite coupling, thereby again binding the further fragment to the root fragment (FIG 7).

Preferably, the protection of one of the further fragment is again removable; which may be performed either while the resulting nucleic acid (analogue) is still attached to the assembly substrate (as shown in FIG 8) or after detaching the nucleic acid (analogue).

Finally, the attachment of the root fragment to the assembly substrate is optionally also here reversible, so that the synthesized nucleic acid (analogue) can be detached from the assembly substrate (FIG 9).

Note that as depicted, the root fragment is again attached to the assembly substrate via its 5' end. However, the opposite orientation is also in this example equally possible, since once more-in contrast to Example 1-the coupling between the root fragment and the further fragment does here also not rely on a difference in reaction kinetics between a free 3' and a free 5' end. Depending on the orientation of the root fragment and the end where the protection is realized, the further fragment can again be synthesized in the forward or reverse direction. Again generally though, because the further fragment and root fragment are typically to be coupled in a 3' to 5' (or 5' to 3') fashion, if the protected end is the end through which the further fragment was attached to the synthesis substrate, the synthesis direction (e.g. 3' to 5') of the further fragment is typically opposite to the orientation (e.g. 5' to 3', the 5' end being attached to the assembly substrate) of the root fragment; and *vice versa.* The above notwithstanding, if providing the further fragment comprises capping erroneous strands to prevent them from growing further, it may again be preferred to protect the end through which the further fragment was attached to the synthesis substrate-and thus to synthesize the further fragment in the direction opposite the orientation of the root fragment. This will result in the erroneous strands being capped off at the non-protected end, thereby rendering these erroneous strands unreactive at both ends and thus preventing them from coupling with a root fragment.

It is to be understood that although preferred embodiments, specific constructions, configurations and materials have been discussed herein in order to illustrate the present invention. It will be apparent to those skilled in the art that various changes or modifications in form and detail may be made without departing from the scope of the invention as defined in the appended claims.

## Claims

1. A method for synthesizing a nucleic acid or nucleic acid analogue, comprising:
a) providing a root fragment of the nucleic acid or nucleic acid analogue,
b) providing a further fragment of the nucleic acid or nucleic acid analogue,
c) phosphitylation of either the root fragment or the further fragment, and
d) binding the further fragment to the root fragment by a phosphoramidite coupling;
wherein the root fragment and the further fragment each have a length of at least 5 monomers, preferably at least 10 monomers, more preferably at least 20, yet more preferably at least 30, still yet more preferably at least 50.

2. The method according to claim 1, wherein a ratio of the length of the further fragment to the length of the root fragment is from 0.8 to 1.2, preferably from 0.9 to 1.1, more preferably from 0.95 to 1.05.

3. The method according to any of the previous claims, wherein in step d the root fragment and the further fragment each have a 3' or 5' end rendered unreactive to phosphoramidite coupling.

4. The method according to claim 2, wherein in step d the root fragment is attached to a substrate.

5. The method according to any of the previous claims, wherein the root fragment and/or the further fragment are synthesized on a substrate; preferably each on a different substrate.

6. The method according to claim 5, wherein the root fragment and/or the further fragment is at one end attached to the substrate by a linker, the linker being such that said fragment can be detached from the substrate while leaving a protective group on said end rendering the end unreactive to phosphoramidite coupling.

7. The method according to any of the previous claims, wherein the root fragment and/or the further fragment-preferably both-are synthesized using a method comprising capping an erroneous fragment to prevent further chain elongation, and wherein providing the root fragment and the further fragment with the end rendered unreactive to phosphoramidite coupling is performed such that erroneous fragments are unreactive to phosphoramidite coupling at both ends.

8. The method according to claim 7, wherein the root fragment and/or the further fragment-preferably both-are synthesized using phosphoramidite synthesis.

9. The method according to any of the previous claims, wherein in step c the root fragment or further fragment is phosphitylated while attached to a substrate.

10. The method according to any of the previous claims, wherein the root fragment or further fragment is phosphitylated using chloro N,N,N',N'-tetraisopropyl phosphorodiamidite.

11. The method according to any of the previous claims, wherein after step d the root fragment extended with the further fragment becomes a new root fragment or a new further fragment, and wherein step a to d are repeated at least 1 time; preferably at least 2 times, more preferably at least 5 times, yet more preferably at least 10 times, most preferably at least 20 times.

12. The method according to any of the previous claims, wherein the root fragment and the further fragment each have a 5' and a 3' end; and wherein in step d
- the root fragment is attached to an assembly substrate through its 5' end and the further fragment comprises a protective group at its 3' end, or
- the root fragment is attached to an assembly substrate through its 3' end and the further fragment comprises a protective group at its 5' end.

13. The method according to claim 12, wherein the root fragment is phosphitylated while attached to the assembly substrate.

14. The method according to claim 12 or 13, wherein the further fragment is synthesized on a synthesis substrate
- in a 3' to 5' direction if the root fragment is in step d attached to the assembly substrate through its 5' end, or
- in a 5' to 3' direction if the root fragment is in step d attached to the assembly substrate through its 3' end.

15. A device for synthesizing a nucleic acid or nucleic acid analogue by the method according to any of the previous claims, comprising:
i) a component configured for receiving the root fragment and the further fragment of the nucleic acid or nucleic acid analogue,
ii) a component configured for phosphitylating either the root fragment or the further fragment, and
iii) a component configured for binding the further fragment to the root fragment by a phosphoramidite coupling.
